# FASCICULE DE BREVET EUROPEEN

(11) **EP 1 019 512 B1**
(45) Date de publication et mention de la délivrance du brevet: **07.02.2007**
(21) Numéro de dépôt: 98947596.7
(22) Date de dépôt: 02.10.1998
(51) Int. Cl.: C12N 15/52, C12N 15/54, C12N 15/82, C12N 5/10, C10M 105/34, A01H 5/00

(54) **PROCEDE DE PRODUCTION D'ACIDES GRAS RAMIFIES AU MOYEN DE PLANTES GENETIQUEMENT MODIFIEES**
VERFAHREN ZUR HERSTELLUNG VERZWEIGTKETTIGER FETTSÄUREN MITTELS GENETISCH MODIFIZIERTER PFLANZEN
METHOD FOR PRODUCING BRANCHED FATTY ACIDS USING GENETICALLY MODIFIED PLANTS

(30) Priorité: 03.10.1997 FR 9712368
(43) Date de publication de la demande: 19.07.2000
(73) Titulaire: Total France, 92800 Puteaux (FR)
(72) Inventeur: DUHOT, Pierre, F-95220 Herblay (FR); GONTIER, Eric, F-54170 Germiny (FR); THOMAS, Daniel, F-60150 Villers-sur-Coudun (FR); THOMASSET, Brigitte, F-60200 Compiègne (FR); MENARD, Marc, F-60200 Compiègne (FR)
(74) Mandataire: Desaix, Anne
(86) Numéro de dépôt international: PCT/FR1998/002116
(87) Numéro de publication internationale: WO 1999/018217

(56) Documents cités:
- WO-A-91/14670
- WO-A-96/03511
- SCHMID K.: "Dihydrosterculate in tobacco transformed with bacterial cyclopropane fatty acid synthase" PLANT LIPID METABOLISM,1995, pages 108-110, XP002068786
- OHLROGGE J.: "Design of new plant products: engineering of fatty acid metabolism" PLANT PHYSIOLOGY, vol. 104, 1994, pages 821-826, XP002068787

## Description

### INTRODUCTION

La présente invention concerne un procédé de production d'acides gras à chaîne aliphatique non linéaire. Le procédé de l'invention réside plus particulièrement dans l'utilisation de plantes modifiées génétiquement. Les acides gras ainsi produits, sous forme libre ou non, éventuellement modifiés par voie chimique ou enzymatique, peuvent être utilisés dans la fabrication de différents produits industriels, notamment de lubrifiants de type huile hydraulique ou huile moteur.

Les acides gras sont des acides à chaîne aliphatique longue généralement comprise entre 6 et 25 atomes de carbone. La chaîne aliphatique peut être linéaire ou non linéaire (également désignée ramifiée). Les acides gras produits naturellement se présentent soit sous forme libre, soit sous forme estérifiée, notamment sous forme de diglycérides ou triglycérides. Les acides gras sont généralement utilisés dans différents types d'industries, notamment pour la préparation de bases lubrifiantes d'origine naturelles ou dans des détergents, adjuvants, ou encore dans la constitution de biocarburants. Pour toutes ces applications, il est particulièrement avantageux de pouvoir utiliser des acides gras d'origine naturelle, notamment dérivés de matière végétale. Ainsi, différents procédés de production et d'extraction d'acides gras à partir de plantes (notamment de graines de plantes) ont été décrits dans l'art antérieur (voir notamment Harrington et al, Ind. Eng. Chem. Prod. Res. Dev. 1985, Vol. 24, page 314; FR 2 722 798). Par ailleurs, la demande WO 94/13814 est relative à des cellules de plantes génétiquement modifiées par une enzyme impliquée dans le transfert des acides gras libres sur le cholestérol. Néanmoins, si cette demande permet de modifier la composition des triglycérides, elle ne permet nullement d'altérer la structure de la chaîne aliphatique des acides gras. Schmid K. (Plant Lipid Metabolism (1995, 108) mentionne la synthèse d'acides gras cycliques (dihydrosterculate) dans des cellules de tabac modifiées génétiquement. Toutefois, ce document ne décrit pas la production d'acide gras ramifiés, ni les moyens correspondants, ni les applications industrielles.

L'un des inconvénients majeurs des procédés antérieurs utilisant des plantes, réside précisément dans la faible diversité des acides gras que l'on peut obtenir. Ainsi, les plantes produisent naturellement essentiellement des acides gras à chaîne aliphatique linéaire de type C6 à C24. Or, les études réalisées par la Société demanderesse ont montré que pour obtenir une bonne base lubrifiante, il était particulièrement avantageux d'utiliser des acides gras à chaîne aliphatique non linéaire (ou ramifiée). Ainsi, la Demanderesse a montré que l'emploi d'acides gras (libres ou non) à chaîne aliphatique ramifiée, permettait d'obtenir des produits de type lubrifiant présentant une bonne stabilité thermique, une bonne résistance à l'oxydation, une bonne viscosité, une bonne biodégradabilité ainsi que des points de fusion (ou points d'écoulement) bas.

Il existe donc un réel besoin de disposer de procédés permettant de produire efficacement des acides gras ramifiés. La présente invention apporte justement une solution avantageuse à ces besoins en décrivant des procédés basés sur la modification génétique de plantes.

Ainsi, un premier aspect de l'invention concerne un procédé de production d'acides gras ramifiés au moyen de plantes génétiquement modifiées.

Un autre aspect de l'invention réside dans des plantes génétiquement modifiées capables de produire des acides gras ramifiés et dans un procédé de préparation de telles plantes. Un autre aspect de l'invention réside également dans des cellules de plantes contenant un acide nucléique recombinant codant pour un produit induisant ou stimulant la synthèse dans ladite cellule d'acides gras ramifiés.

La présente invention est également relative à l'acide nucléique recombinant tel que défini ci-dessus et à tout vecteur le comportant.

### DESCRIPTION DÉTAILLÉE DE L'INVENTION.

La présente invention est donc relative à un procédé de production d'acides gras ramifiés à partir de plantes génétiquement modifiées, à des plantes et cellules de plantes génétiquement modifiées produisant des acides gras ramifiés, ainsi qu'à des acides nucléiques recombinants et des vecteurs les contenant, utilisables à cet effet.

Au sens de l'invention, on entend par acide gras ramifié, tout acide gras possédant une chaîne aliphatique ramifiée vraie. Comme il ressort du texte de la présente demande, le terme "ramifié" désigne au sens de l'invention des acides gras portant une ou plusieurs substitutions, sur une ou plusieurs positions distinctes de la chaîne aliphatique. Ainsi, la seule présence d'un cycle dans la chaîne aliphatique ne suffit pas à qualifier l'acide gras de ramifié selon l'invention. Il s'agit préférentiellement d'un acide gras possédant une chaîne aliphatique en C6 à C24, encore plus préférentiellement en C12 à C24. Des exemples de tels acides, essentiellement sous forme saturée et non encore ramifiée sont notamment l'acide caprique (C10), le laurique (C12), le myristique (C14), le palmitique (C16), le stéarique (C18), l'éicosanoïque (C20), le docosanoïque (C22), le tétracosanoïque (C24) et des mélanges de ceux-ci. La chaîne aliphatique des acides gras ramifiés de l'invention peut être ramifiée en une ou plusieurs positions par différents groupements. Avantageusement, la chaîne aliphatique comporte une à quatre ramifications localisées dans la partie centrale de la chaîne aliphatique et/ou dans sa partie terminale opposée à la fonction acide. Plus préférentiellement, la ou les ramifications sont localisées sur un ou plusieurs carbone compris entre les positions 8 et 15 de la chaîne aliphatique. Par ailleurs, le groupement ramifié peut être tout groupe alkyl, de préférence un alkyl en C1 à C5, encore plus préférentiellement un méthyle, éthyle, propyl, isopropyl, butyle, terbutyl. Un groupement ramifié particulièrement préféré est un groupement méthyle. Des acides gras ramifiés particuliers au sens de l'invention sont par exemple l'acide méthyl 9 octadécanoïque ; l'acide méthyl 10 octadécanoïque ; l'acide diméthyl 9,12 octadécanoïque ; l'acide diméthyl 10,12 octadécanoïque; l'acide diméthyl 9,13 octadécanoïque; l'acide diméthyl 10,13 octadécanoïque ; l'acide diméthyl 9,12 oléique ; l'acide diméthyl 10,12 oléique; l'acide diméthyl 9,13 oléique ; l'acide diméthyl 10,13 oléique ou encore l'acide 2, 4, 6, 8-tétraméthytdécanoique.

Par ailleurs, les acides gras ramifiés selon l'invention peuvent être des acides gras ramifiés sous forme libre ou sous forme de dérivés. Il peut s'agir notamment d'esters d'acide gras ramifié, tels que des monoesters, diesters ou triesters du glycérol (triglycéride). Dans le cas de di- ou triglycérides, il n'est par ailleurs pas nécessaire que la totalité des acides gras présents dans la molécule soit ramifiée. Ainsi, un triglycéride peut comporter 1, 2 ou 3 acides gras ramifiés. Il est toutefois préférable que la proportion d'acides gras ramifiés dans les esters ou autres dérivés soit élevée.

Un premier objet de l'invention est plus particulièrement relatif à un procédé de production d'acides gras ramifiés, caractérisé en ce que lesdits acides gras ramifiés sont produits à partir d'au moins une cellule végétale ou d'un matériel végétal ou d'une plante comprenant au moins une cellule végétale, ladite cellule végétale comprenant dans son génome un acide nucléique recombinant codant pour un produit induisant ou stimulant la synthèse d'acide(s) gras ramifié(s).

On désigne au sens de l'invention par "acide nucléique recombinant", tout acide ribonucléique ou désoxyribonucléique construit par les techniques du génie génétique. Il peut s'agir en particulier d'un ADN complémentaire, d'un ADN génomique ou d'un ARN messager. Un tel acide nucléique peut par ailleurs être d'origines variées, notamment bactérienne, virale, végétale, mammifère, synthétique ou semi-synthétique. Un acide nucléique recombinant peut être préparé en utilisant les techniques connues de l'homme du métier, et notamment par criblage de banques d'acides nucléiques avec des sondes appropriées, par synthèse chimique (en utilisant des synthétiseurs d'acides nucléiques), par digestions/ligatures enzymatiques, etc. ou toute combinaison de ces méthodes.

La présente invention peut être mise en oeuvre avec différents types de plantes, de préférence des plantes cultivables en champs. Il s'agit avantageusement d'une plante oléagineuse, telle que par exemple le colza, le tournesol, l'arachide, le soja, le lin (oléagineux) ou encore le maïs. Il peut également s'agir d'une plante de type tabac.

Les cellules de plantes selon l'invention peuvent être toute cellule de plantes telles que définies ci-avant. Il peut s'agir plus préférentiellement d'une cellule de plante cultivable, et capable de régénérer une plante. Le terme "matériel végétal" désigne toute partie d'une plante telle que définie ci-dessus, comprenant au moins une cellule. Il peut s'agir de tout tissu de ladite plante, tel que notamment de graines.

La cellule ou plante selon l'invention est donc génétiquement modifiée, de sorte qu'elle comprend dans son génome sous forme libre ou intégrée, un acide nucléique recombinant codant pour un produit induisant ou stimulant la synthèse d'acide gras ramifié. Les plantes et cellules de plantes de l'invention comportent donc des acides gras dont une fraction est constituée d'acides gras ramifiés. Selon les applications envisagées, il peut être avantageux que cette fraction représente une proportion substantielle des acides gras totaux. Avantageusement, l'acide nucléique recombinant est présent dans la cellule sous forme intégrée au génome de ladite cellule, de manière à être plus stable ségrégationellement.

Un produit est désigné comme induisant la synthèse d'acide gras ramifié lorsqu'il permet à la cellule de plante le contenant d'effectuer une synthèse qu'elle n'aurait pu réaliser en son absence. On parle d'un produit stimulant la synthèse d'acide gras ramifié, lorsqu'il s'agit d'un produit qui permet à la cellule de plante le contenant de favoriser une voie de synthèse préexistante dans ladite cellule mais non ou peu exploitée par celle-ci, par exemple pour des raisons de stoechiométrie ou de spécificité de substrat. L'acide nucléique recombinant utilisé dans la présente invention, peut coder plus particulièrement, pour un produit capable d'induire une ramification des acides gras produits par la cellule post-synthèse ou pour un produit permettant d'effectuer une ramification concomitante à ladite synthèse. Ces deux approches sont par ailleurs combinables.

Les acides gras sont synthétisés par les cellules de plantes dans le chloroplaste. Cette synthèse comprend plusieurs cycles répétitifs au cours desquels des unités carbonées sont ajoutées successivement les unes aux autres pour générer la chaîne aliphatique grasse. Après leur synthèse, ces acides gras linéaires sont exportés vers le cytoplasme où ils subissent différentes modifications post-synthèse (ou post-élongation) et notamment une désaturation par des enzymes désignés désaturases.

Dans un premier mode de réalisation, l'acide nucléique recombinant code pour un produit permettant d'induire ou de stimuler la ramification post-synthèse des acides gras produits par ladite cellule de plante. Plus particulièrement, selon ce premier mode de réalisation de l'invention, l'acide nucléique recombinant code pour une enzyme par exemple, une méthyle transférase ou une cyclopropane fatty acide synthase, permettant le transfert d'un ou plusieurs groupements alkyl sur la ou les doubles liaisons des acides gras insaturés.

Les groupements alkyl transférés peuvent être, comme indiqué ci-avant, tout groupement alkyl comportant de 1 à 5 atomes de carbone préférentiellement. Il s'agit avantageusement de groupements alkyl comprenant de 1 à 3 atomes de carbone et notamment de groupement méthyle, éthyle, propyl ou isopropyl.

Avantageusement, l'acide nucléique recombinant code pour une enzyme hétérologue, c'est-à-dire une enzyme issue d'un organisme autre qu'une cellule de plante. Plus particulièrement, l'enzyme utilisée provient avantageusement d'un microorganisme procaryote ou eucaryote telle que par exemple une bactérie ou une levure.

On peut citer à titre illustratif des acides nucléiques recombinants selon l'invention un acide nucléique recombinant codant pour la cyclopropane fatty acide synthase encore désignée CFAS. L'acide nucléique peut également coder pour des méthyl transférase tel que par exemple des SAM-méthyl transférase c'est-à-dire des enzymes capables de catalyser le transfert d'un groupement méthyle depuis la SAM (S-Adénosyl-Méthionine) vers un acide gras insaturé ou plus généralement vers une double liaison d'une chaîne aliphatique. Un acide nucléique recombinant particulier au sens de l'invention code par exemple pour la cyclopropane fatty acide synthase telle que décrite par Wang et al (Biochemistry 31 (1992) 11020) ou pour toute enzyme analogue à cette dernière. On entend au sens de la présente invention par le terme "analogue" toute enzyme possédant une ou plusieurs modifications structurales par rapport à l'enzyme décrite ci-avant et conservant une activité de transfert de groupement alkyl sur les doubles liaisons de chaîne aliphatique. Ces modifications structurales peuvent être des mutations, des délétions, des substitutions ou des additions d'un ou plusieurs acides aminés. Le terme "analogue" comprend également les séquences homologues obtenues à partir d'autres organismes. Un autre "analogue" est une enzyme produite par un acide nucléique hybridant avec tout ou partie de la séquence de la transférase ou la synthase et conservant une activité de même nature. L'hybridation est avantageusement réalisée dans des conditions de stringence normale, ou plus préférentiellement forte. Des conditions d'hybridation stringentes sont par exemple : Hybridation à 42°C, 50% formamide, 5 X SSC, 1 X Denhardt ; Lavage à 65°C en 0,1 X SSC, 0,1% SDS. Des conditions d'hybridation non-stringentes sont par exemple : Hybridation à 37°C, 40% formamide, 5 X SSC, 1 X Denhardt ; Lavage à 50°C en 1 X SSC, 0,1% SDS. Les conditions stringentes sont particulièrement adaptées lorsque l'acide nucléique test est présent en faibles quantités et/ou sous forme peu purifiée. Les conditions non stringentes sont plus adaptées lorsque l'acide nucléique test est présent en quantités plus importantes et se trouve sous forme significativement représentée dans l'échantillon. D'autres conditions d'hybridation sont bien connues de l'homme du métier (voir notamment Maniatis et al). Un autre exemple particulier d'acides nucléiques recombinants selon l'invention est un acide nucléique recombinant codant pour la méthyl-transférase responsable de la synthèse de l'acide 9-méthyl-10-hexadecénoïque chez Corynebacterium spp. (Niepel et al., J. Bact. 180 (1998) 4650) ou dans une variété d'algue (Carballeira et al., Lipids 32 (1997) 1271).

Par ailleurs, ce mode de réalisation consistant à transférer un groupement alkyl sur une chaîne aliphatique fait appel aux groupements alkyl ou donneurs d'alkyl présents dans ladite cellule. A cet égard, pour le transfert d'un groupement méthyl, les réserves de la cellules sont principalement constituées par le groupement SAM. Aussi, dans le but d'améliorer encore l'efficacité de cette réaction, et donc le rendement en acides gras ramifiés, un mode de réalisation particulier consiste à introduire dans la cellule de plante, en plus de l'acide nucléique codant pour le produit défini ci-dessus, un second acide nucléique codant pour une enzyme impliquée dans la synthèse du donneur d'alkyl. Préférentiellement, il s'agit d'un acide nucléique codant pour une enzyme de synthèse du SAM. Encore plus préférentiellement, il s'agit d'un acide nucléique codant pour la SAM synthétase ou un analogue de celle-ci. Cet acide nucléique peut être introduit par l'intermédiaire d'un second acide nucléique recombinant, de manière simultanée avec le premier ou séquentiellement. Selon un autre mode de mise en oeuvre, les acides nucléiques codant pour les deux enzymes sont portés par le même acide nucléique recombinant.

Selon un deuxième mode de réalisation de la présente invention, l'acide nucléique recombinant contenu dans la cellule de plante, code pour un produit permettant d'induire ou de stimuler l'incorporation dans la chaîne aliphatique d'acide gras en cours d'élongation, de groupements générant une ou des ramifications. Selon cette deuxième approche, il ne s'agit donc pas d'effectuer une modification post-synthèse sur les acides gras produits mais d'effectuer une ramification de manière concomitante à ladite synthèse.

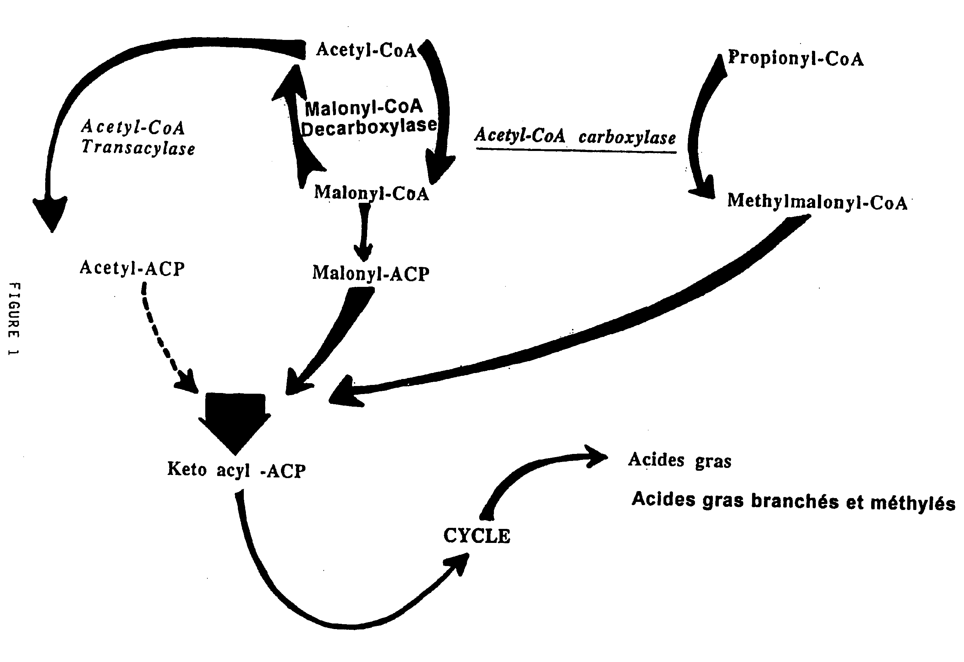

Ce mode de réalisation est préférentiellement obtenu en forçant la cellule de plante à utiliser, comme substrat pour la synthèse de la chaîne aliphatique, un groupement Acyl-CoA comportant un nombre d'atomes de carbone supérieur à 3, de préférence entre 4 et 8 à la place du malonyl CoA. Avantageusement, le substrat utilisé est un groupement Acyl-CoA non linéaire. Encore plus préférentiellement, il s'agit d'un groupement Acyl-CoA non linéaire permettant l'incorporation dans la chaîne aliphatique en cours d'élongation d'un groupement à nombre impair d'atomes de carbone. Cette stratégie permet par exemple d'obtenir des acides gras ramifiés dits multibranchés, tels que par exemple l'acide 2, 4, 6, 8-tétraméthyldécanoique. Un exemple particulier de substrat de type Acyl-CoA est par exemple le méthylmalonyl CoA. Le méthylmalonyl CoA est un groupement non linéaire, comportant 4 atomes de carbone, dont trois servent à l'élongation. Dans ce cas, dans le but de forcer la plante à utiliser ce substrat pour la synthèse de la chaîne aliphatique, il est particulièrement avantageux d'introduire dans la cellule de la plante un acide nucléique recombinant codant pour une malonyl CoA décarboxylase (E.C. N° 4.1.1.9). Le mode d'action de cette enzyme ainsi que de manière plus générale la voie de synthèse des acides gras ramifiés par les cellules modifiées selon cette stratégie est représenté sur la figure 1. Il est entendu que tout autre enzyme analogue à celle mentionnée ci-dessus et capable de forcer la plante à utiliser un acyl-CoA comportant plus de 3 atomes de carbone, de préférence 4 à 8, comme substrat dans la synthèse des chaînes aliphatiques des acides gras, peut être utilisée dans la présente invention. A titre d'exemple particulier de telles enzymes, on peut citer notamment la malonyl CoA décarboxylase dont la séquence nucléotidique a été décrite dans la littérature (Cf les exemples), ainsi que tout analogue de celle-ci.

Ce deuxième mode de réalisation peut également être obtenu en modifiant la spécificité des enzymes impliquées dans la synthèse de la chaîne aliphatique grasse, notamment en modifiant la spécificité de l'enzyme qui empêche le transfert du malonyl CoA sur le malonyl ACP, et en particulier la spécificité de tout ou partie de la Fatty Acid Synthase, de l'acétyl CoA carboxylase ou de l'acétyl CoA transacylase. A cet égard, la Fatty Acid Synthase est constituée d'un complexe multi-enzymatique et la modification de sa spécificité peut être obtenue par modification d'une, plusieurs, voire toutes les sous-unités.

Par ailleurs, comme indiqué ci-avant, ces deux modes de réalisation peuvent être combinés pour générer des cellules de plantes capables de synthétiser des acides gras à chaîne aliphatique ramifiée à la fois au stade de l'élongation et post-élongation.

Pour la mise en oeuvre de la présente invention, l'acide nucléique recombinant comporte avantageusement des régions régulatrices, de type promoteur, fonctionnelles dans les cellules de plantes et permettant de contrôler l'expression des acides nucléiques définis ci-dessus. Parmi les régions régulatrices de la transcription utilisables dans les plantes et dans la mise en oeuvre de la présente invention, on peut citer par exemple, les régions associées au virus de la mosaïque du chou-fleur (35S, 19S) ou les régions promotrices de gènes structuraux tels que les gènes de la nopaline synthase (nos) ou l'octopine synthase (ocp) ou la mannopine, l'agropine ou l'acyl carrier protein (ACP). La structure et le clonage de ces différentes régions ont été décrites dans la littérature. De manière plus générale, tout promoteur permettant une expression constitutive, spatiale ou temporelle d'un acide nucléique dans une cellule de plante peut être utilisé pour la mise en oeuvre de la présente invention. A cet égard, il est possible notamment d'utiliser des promoteurs permettant une expression localisée à certains tissus ou parties de la plante (graine, pollen, etc.), ou des promoteurs dont l'activité dépend du stade de développement de la plante (élongation, floraison, etc.). Comme promoteur ayant une expression majoritaire dans les graines on peut citer par exemple le promoteur de l'ACP, de la napine ou les promoteurs décrits par Krebbers et al. (Plant Physiol. 87 (1988) 859). L'emploi de tels promoteurs peut éviter tout effet secondaire potentiel induit par une production massive et généralisée d'acides gras ramifiés sur la plante, et également faciliter la récupération des acides gras ainsi produits, en les concentrant dans certaines régions de la plante.

Par ailleurs, il est également avantageux d'introduire dans les acides nucléiques recombinants selon l'invention, des séquences terminatrices localisées en 3' qui peuvent dériver soit directement du gène utilisé ou provenir de régions d'autres gènes, tels que ceux notamment de la nopaline synthase (nos) ou encore de l'octopine synthase (ocp) de la mannopin, de l'agropine ou de l'ACP.

L'acide nucléique recombinant qui comporte donc avantageusement une région régulatrice de la transcription, un gène ou une région codant pour un produit actif tel que défini ci-avant, et des régions terminatrices en 3', est avantageusement introduit dans un vecteur plasmidique de clonage ou d'expression.

A cet égard, un autre objet de l'invention concerne plus particulièrement un acide nucléique recombinant comprenant:
- un acide nucléique codant pour un produit tel que défini ci-avant,
- un promoteur contrôlant l'expression dudit acide nucléique, et capable de causer une expression de cet acide nucléique localisée à certains tissus végétaux ou certaines parties végétales, et,
- une région 3' de terminaison de la transcription.

Plus particulièrement, le promoteur compris dans les constructions génétiques de l'invention est un promoteur capable de causer une expression de l'acide nucléique localisée dans la graine de la plante. Le terme "expression localisée" signifie que l'expression est nettement plus importante dans le tissu concerné que dans les autres tissus végétaux. Il est entendu néanmoins qu'une spécificité absolue d'expression n'est pas requise, dès lors qu'une expression sigificative est observée dans les tissus recherchés. Le caractère localisé de l'expression selon l'invention est doublement avantageux puisqu'il facilite d'une part la récupération des acides gras ramifiés et qu'il limite les risques de toxicité (notamment sur la bonne croissance et la reproduction) des produits exprimés sur les plantes modifiées.

Les acides nucléiques recombinants de l'invention peuvent par ailleurs comporter des signaux d'adressage, permettant d'améliorer l'adressage des protéines synthétisées vers les compartiments cellulaires où elles exercent leur activité. Lorsque le produit synthétisé est un produit impliqué dans le transfert de groupements alkyl sur des chaînes grasses post-élongation, la protéine exerce son activité dans le cytoplasme et ne nécessite donc pas de signaux d'adressage particuliers. Lorsque le produit intervient sur la synthèse même des chaînes grasses, il exerce son activité au sein des chloroplastes et il peut être avantageux d'utiliser un signal permettant l'adressage de ce produit dans le chloroplaste. De tels signaux sont connus dans l'art antérieur.

Un autre objet de l'invention est relatif à un acide nucléique recombinant comprenant:
- un acide nucléique codant pour une méthyl-transférase capable de catalyser le transfert d'un groupement méthyle vers la chaîne aliphatique d'un acide gras insaturé,
- un promoteur fonctionnel dans les cellules végétales contrôlant l'expression dudit acide nucléique, et,
- une région 3' de terminaison de la transcription.

Dans un autre mode de réalisation particulier, l'invention a pour objet un acide nucléique recombinant comprenant:
- un acide nucléique codant pour une malonyl CoA décarboxylase,
- un promoteur fonctionnel dans les cellules végétales contrôlant l'expression dudit acide nucléique, et,
- une région 3' de terminaison de la transcription.

Un autre objet de la présente invention est également relatif à un vecteur comportant un acide nucléique recombinant tel que défini ci-avant. Un tel vecteur peut notamment être un dérivé du vecteur Ti. Les vecteurs de l'invention peuvent être utilisés pour produire des quantités importantes d'acides nucléiques recombinants dans les cellules, ou pour produire les enzymes correspondantes, ou pour transformer des cellules végétales.

La présente invention est également relative à toute cellule de plante comprenant un acide nucléique recombinant ou un vecteur tels que définis ci-avant.

Les acides nucléiques recombinants ou vecteurs de l'invention peuvent être introduits dans les plantes par toute technique connue de l'homme du métier, et notamment par des techniques chimiques, physiques ou biologiques. Les techniques chimiques impliquent par exemple l'emploi d'agents transfectants facilitant la pénétration cellulaire. Les techniques physiques sont par exemple l'électroporation, le bombardement, les "gene guns", etc. Une technique biologique bien connue et particulièrement efficace consiste à utiliser des infiltrations sous vide ou co-culture, vecteurs viraux ou, préférentiellement, Agrobactérium tumefaciens, qui permet d'introduire du matériel génétique dans les cellules de plantes au moyen du plasmide Ti. Ces techniques sont bien connues de l'homme du métier. Après transformation, les cellules de plantes contenant effectivement l'acide nucléique selon l'invention sont sélectionnées. Ces cellules peuvent alors être maintenues en culture, éventuellement multipliées en culture, et utilisées directement pour la production d'acides gras ramifiés (cultures en batch, fed batch, etc.). Ces cellules peuvent également être stockées en vue d'une utilisation ultérieure. Ces cellules peuvent également être utilisées pour régénérer des plantes transgéniques, selon les techniques classiques de la biologie végétale.

Un autre objet de l'invention porte donc sur un procédé de préparation d'une plante transgénique capable de produire des acides gras ramifiés, comprenant l'introduction d'un acide nucléique recombinant tel que défini ci-avant dans une cellule ou partie de plante et la régénération, à partir de ces cellules ou parties de plantes d'une plante transgénique. Avantageusement, l'introduction est réalisée en utilisant Agrobactérium. Dans un mode particulier, l'introduction est réalisée sur des cellules de plantes ou des disques foliaires. La régénération est effectuée par toute technique connue de l'homme du métier.

Un autre objet de l'invention porte sur une plante transgénique dont les cellules contiennent un acide nucléique recombinant tel que décrit ci-dessus et codant pour les enzymes permettant d'induire ou de stimuler la ramification post-synthèse des acides gras produits par ladite cellule de plante, ou des séquences permettant d'induire ou de stimuler l'incorporation dans la chaîne aliphatique d'acide gras en cours d'élongation, de groupements ramifiés.

Les plantes transgéniques de l'invention contiennent l'acide nucléique recombinant intégré de façon stable dans leur génome, tant dans celui des cellules germinales que celui des cellules somatiques.

L'invention concerne également tout matériel végétal issu d'une telle plante, et notamment les graines.

L'invention est également relative à un procédé de production d'acides gras ramifiés par culture d'une cellule telle que définie ci-avant et récupération des acides gras produits.

L'invention concerne également un procédé de production d'acides gras ramifiés par culture cellulaire d'une cellule de plante comprenant :
- la culture de ces cellules dans un milieu approprié à leur croissance,
- l'extraction et la purification des acides gras ramifiés à partir de la biomasse cellulaire, et notamment à partir de ces cellules ou du surnageant de ladite culture.

L'invention concerne en outre un procédé de préparation d'acides gras ramifiés par extraction à partir d'une plante transgénique dont les cellules contiennent un acide nucléique recombinant tel que défini ci-dessus, selon lequel lesdits acides gras sont récupérés à partir de toute partie de ladite plante, et notamment des graines de ladite plante.

L'invention concerne un procédé de préparation d'acides gras ramifiés à partir d'une plante transgénique dont les cellules contiennent un acide nucléique recombinant comprenant :
- la culture en champs desdites plantes transgéniques;
- la récupération des graines desdites plantes ;
- l'extraction des acides gras à partir de ces graines.

Le procédé de l'invention est tout particulièrement adapté à la production d'acides gras méthylés non-cycliques, notamment d'acides gras en C16 ou C18 portant un ou plusieurs groupements méthyle. A titre d'exemples préférés, on peut citer les acides méthyl-9 octadécanoïque, méthyl-10 octadécanoïque, méthyl 9-hexadécanoïque et méthyl 10-hexadécanoïque.

Dans les procédés de production d'acides gras ramifiés tels que définis ci-avant, une étape facultative de traitement des acides gras peut être pratiquée, afin d'améliorer encore les propriétés physicochimiques de ces composés. Ainsi, les procédés de l'invention comprennent notamment:
- une étape d'exrtraction des acides gras ramifiés à partir des cellules, graines ou plantes, et,
- une étape de traitement des acides gras pour améliorer leurs propriétés physicochimiques.

L'extraction peut être réalisée par les méthodes classiques connues de l'homme du métier citées plus haut.

Plus préférentiellement, l'étape de traitement est une étape d'hydrogénation, réalisée dans des conditions standard ou fortes, conduisant à la formation d'acides gras ramifiés saturés. Plus spécifiquement, l'hydrogénation peut être réalisée dans les conditions décrites par Christie W.W. (Topics in Lipid Chemistry, 1970, Vol. 1, FD Gunstone, ed. London : Logus Press), incorporé à la présente par référence, le cas échéant en présence d'un catalyseur au palladium.

L'invention concerne plus généralement l'utilisation d'une plante transgénique régénérée à partir d'une cellule telle que définie ci-avant, pour la production d'acides gras ramifiés.

L'invention concerne également l'utilisation d'une plante ou partie d'une plante transgénique dont au moins certaines des cellules comprennent un acide nucléique recombinant tel que défini ci-avant, pour la production d'acides gras ramifiés.

Les inventeurs ont notamment montré que les esters d'acides gras ramifiés tels que définis dans l'invention avaient une bonne résistance à l'oxydation et des points d'écoulement bas, et possédaient donc des propriétés avantageuses pour une utilisation comme lubrifiants.

Les esters d'acides gras ramifiés tels que définis ci-avant peuvent être utilisés comme lubrifiants, notamment pour la préparation de compositions d'huile, en particulier d'huile moteur et d'huile hydraulique, notamment en remplacement des bases non-conventionnelles ou synthétiques (telles que les esters, polyalphaoléfines, etc.).

Une composition lubrifiante peut comprendre des acides gras ramifiés tels que décrits ci-avant, et un ou plusieurs additifs, tels que notamment des agents anti-usure, des modificateurs de pression, des tensioactifs, etc.

La présente demande sera décrite plus en détails à l'aide des exemples qui suivent, qui doivent être considérés comme illustratifs et non limitatifs.

### LÉGENDE DES FIGURES

Figure 1 : Schéma de la synthèse des acides gras dans le chloroplaste des cellules de plante
Figure 2: Représentation d'un acide nucléique recombinant comprenant un gène codant pour une transférase sous contrôle du promoteur CaMV 35S (Figure 2A) ou nos (Figure 2B).
Figure 3: Représentation d'un acide nucléique recombinant comprenant un gène codant pour la SAM synthase.
Figure 4: Carte de restriction du gène cfa
Figure 5: Représentation d'une cassette de régulation d'un gène capable de stimuler la production d'acide gras ramifiés.
Figure 6 : Schéma de construction de la cassette de régulation du gène cfa.
Figure 7 : Construction du vecteur pTDEcfa.
Figure 8 : Transformation des disques foliaires et régénération des plantes. A : Coculture agrobactéries-disques foliaires ; B : 2ème semaine de culture ; C : 5ème semaine de culture ; D : enracinement de bourgeons ; E : plantule bouturée in vitro.
Figure 9 : Représentation d'un acide nucléique recombinant comprenant un gène codant pour la malonyl-CoA décarboxylase.

### EXEMPLE A : CONSTRUCTION D'UNE CELLULE DE PLANTE GENETIQUEMENT MODIFIEE CAPABLE DE PRODUIRE DES ACIDES GRAS RAMIFIES PAR ALKYLATION POST-ELONGATION

Cet exemple illustre la construction de plantes génétiquement modifiées capables de produire des acides gras ramifiés par alkylation post-élongation, plus particulièrement par méthylation post-élongation. En particulier, cet exemple décrit l'insertion par génie génétique dans le génome de la plante d'un gène ou d'un ensemble de gènes codant pour :
. d'une part, une ou des transférases, par exemple des méthylases ou des méthyltransférases (de type Cyclopropane Fatty Acide synthase i.e. CFA synthase, par exemple) capable de catalyser l'addition des groupements méthyles (ou autres alkyls : éthyle, propyl, isopropyl...) sur les doubles liaisons d'acides gras insaturés; et,
. d'autre part, d'un ou plusieurs gènes permettant d'augmenter le pool de groupements donneurs de méthyle (S-Adénosyl Méthionine i.e. SAM), notamment du gène codant pour la S-Adénosyl Méthionine synthétase (SAM synthétase).

La construction de ces plantes transgéniques selon l'invention est effectuée selon les étapes suivantes.

### ETAPE 1 : MATÉRIEL VÉGÉTAL UTILISÉ

Les plantes modèles de tabac de type SR1 (Horsch et al., 1985 Science, **22,** 1229-1231) ou BY2 (Nagata et al., 1992 Int. Rev. Of Cytol., **132,** 1-30) sont utilisées, dans un premier temps, pour réaliser les expérimentations. En effet, ces deux espèces modèles sont facilement transformables par *Agrobacterium tumefaciens* (Van Lijsebettens et al., 1986, J. Mol. Biol., **188,** 129-145 ; Shaul et al. 1986; Shaul et al., 1996 Proc. Natl. Acad. Sci. USA, **93,** 4868-4872) et permettent la régénération de nouvelles plantes. Toutes plantes oléagineuses telles que colza, tournesol, arachide, soja, maïs, etc. peuvent bien entendu être utilisée de la même manière.

### ETAPE 2: CONSTRUCTIONS GÉNIQUES.

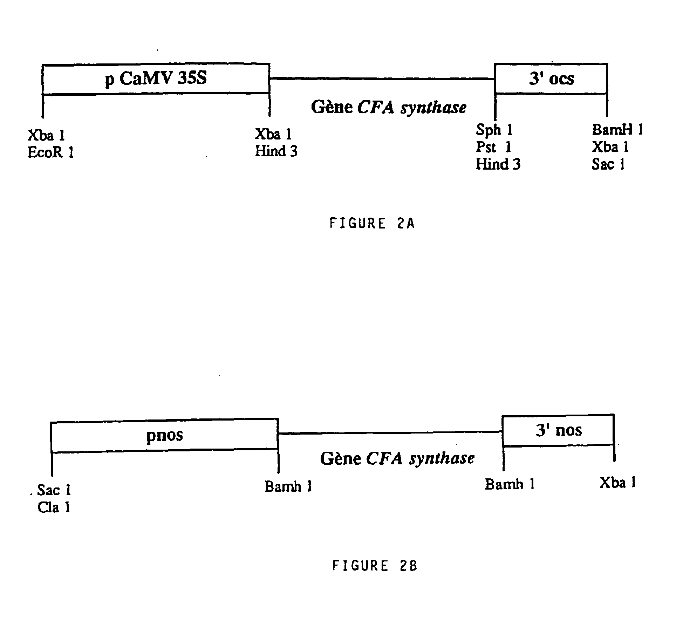

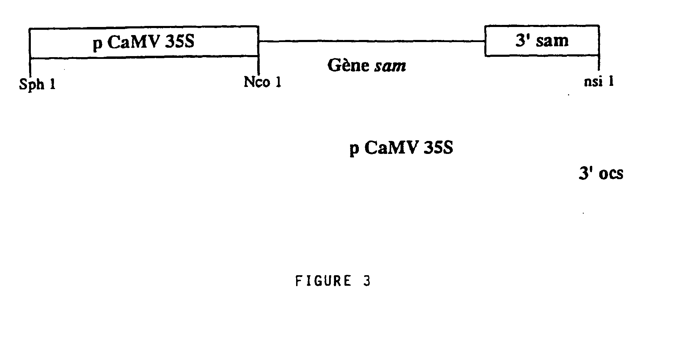

La transformation génétique consiste à introduire un acide nucléique recombinant comprenant une cassette d'expression comprenant le gène à transférer, le promoteur d'expression de ce gène qui pourra permettre une expression constitutive, spatiale ou temporelle, et une région 3' de terminaison de la transcription. Dans ce qui suit sont décrits les différents éléments génétiques utilisés et la construction des acides nucléiques recombinants correspondants (Figures 2 et 3).

### 2.1. Sélection et isolement des acides nucléiques codant pour les enzymes.

Dans cet exemple sont utilisés des acides nucléiques codant pour des méthylases ou des méthyltransférase issue d'organismes procaryotes ou eucaryotes et dont les séquences codantes sont des analogues de la séquence codante de la Cyclopropane Fatty Acide Synthase décrite par Wang et al. (1992 Biochemistry, **31,** 11020-11028). Plus particulièrement, un acide nucléique codant pour la Cyclopropane Fatty Acide Synthase est isolé et sa séquence vérifiée.

Par ailleurs, pour améliorer l'efficacité du procédé, dans un mode de réalisation particulier, un deuxième acide nucléique peut être utilisé, codant pour la SAM synthétase dont la séquence nucléotidique est décrite par Peleman et al. (1989 Plant Cell, **1**, 81-93).

### 2.2. Sélection des régions promotrices

Pour la construction des acides nucléiques recombinants, différents types de promoteurs peuvent être utilisés, et en particulier tout promoteur fonctionnel dans les plantes et permettant le contrôle de l'expression des gènes.

A cet égard, on peut citer les promoteurs permettant une expression localisée dans les graines (ou certains tissus des graines) de plantes, tels que notamment le promoteur du gène codant pour la prolamine (Zhou et al, Transgenic Res. 2 (1993) 141), le promoteur du gène codant pour la lectine de pois (Pater et al., Plant J. 6 (1994) 133), le promoteur du gène codant pour les LEA ("Late Embryogenesis Abundant protein") (Goupil et al., Plant. Mol. Biol. 18 (1992) 1049), le promoteur du gène codant pour la famille des protéines napine (NAP) (Boutilier et al., Plant. Mol. Biol. 26 (1994) 1711), le promoteur du gène codant pour la gluterine du riz (Zhao et al., Plant. Mol. Biol. 25 (1994) 429), le promoteur du gène codant pour l'oléosine (Keddie et al., Plant. Mol. Biol. 19 (1992) 443), le promoteur du gène codant pour la famille S des protéines de stockage (promoteur 2S du gène napA, promoteur 11S ou 12S du gène de la globuline), le promoteur du gène codant pour la béta-phaseoline, la légumine, la conglutine gamma, la concanavaline A, la désaturase Bn10 (Plant. Physiol. 104, 1167), l'alpha/béta gliandine de blé, la catalase CatA du riz, l'alpha-kafirine du sorgo ou encore l'Adh1 du maïs (Kyozuka et al., Plant Cell 6 (1994) 799) ou la protéine SBP65 du pois (Dehaye et al., Plant Mol. Biol. 65 (1997) 605).

Dans cet exemple, les promoteurs utilisés sont le promoteur 35S du virus de la mosaïque du chou fleur et le promoteur du gène de la nopaline synthase (nos): La structure de ces promoteurs a été décrite par exemple par Benfey et Chua (1990, Science, 250 959-966).

### 2.3. Sélection des régions terminatrices de la transcription.

Dans cet exemple, les régions terminatrices de la transcription utilisées proviennent soit directement du gène utilisé (Figure 3), soit des gènes de la nopaline synthase (nos) et de l'octopine synthase (ocs).

### 2.4. Construction des acides nucléiques recombinants (vecteurs).

Pour construire les acides nucléiques recombinants permettant d'introduire les éléments ci-dessus dans les cellules de plantes, ces éléments génétiques sont tout d'abord isolés et sous clonés dans des vecteurs de clonage de type pBR322 et pUC, qui portent des marqueurs permettant la sélection des transformants qui procurent une résistance à des agents cytotoxiques tels que antibiotiques, toxines, etc. Les cassettes d'expression sont ensuite introduites dans des vecteurs binaires contenant les séquences nécessaires à la transformation des plantes, en particulier, le vecteur T-DNA. Ce vecteur est utilisé pour la transformation des plantes et la structure des vecteurs contenant les séquences spécifiques de reconnaissance (RB et LB) a été largement décrite dans la littérature (Livre de Hoekema, the binary Plant Vector Systems, Offsetdrukkerij Kanters B.V., Alblasserdam, 1985. Molecular genetics of the Bacteria-Plant Interaction, Puhler A. Ed., Springer-Verlag, NY, 1983. Plant Genetic Transformation and Gene expression : A Laboratory Manual Draper J., Scott R., Armitage P., et Walden R. Eds, Blackwell Scientific Publications, Oxford, 1988. Kahl G. et Weising K., (1993) Genetic engineering of plant cells. Dans Biotechnology : Genetics Fundamentals and genetic Engineering. Rehm H.J., Reed G., Pühler A. et Stadler P. Eds, VCH Publishers Inc., New York (USA) Vol 2, pp 547-659).

La structure des acides nucléiques recombinants ainsi préparés est représentée sur les Figures 2 et 3.

Dans un autre exemple particulier, le vecteur binaire pTDEcfa a été construit comme suit.

### a) Clonage et caractérisation du gène cfa codant pour la cyclopropane fatty acide synthase.

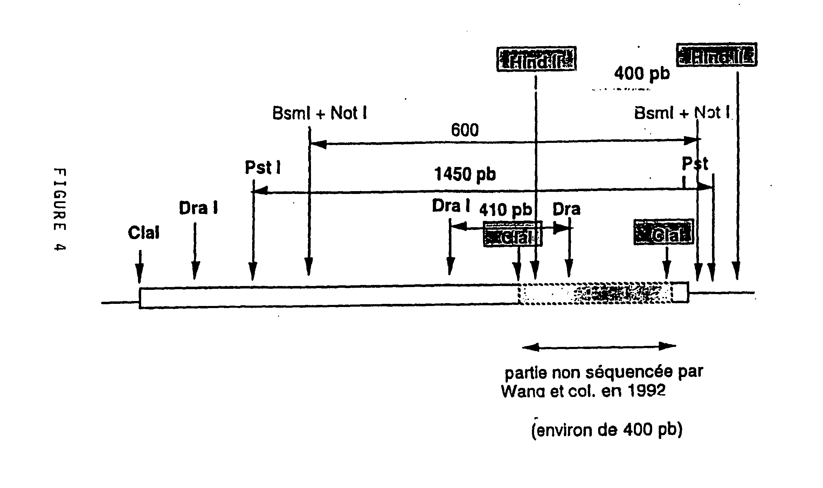

A la lecture des travaux de Wang (Wang et al., 1992) une ambiguïté a été notée concernant la taille du fragment Clal présent dans le plasmide pAYW19. De plus, Wang et al. (1992) présente, dans la stratégie de séquençage, le fragment Clal avec un site unique de restriction pour l'enzyme HindII en amont du site ClaI. Or, d'après la séquence publiée ce site apparaît en aval du site ClaI. Dans une première étape, il a donc été entrepris de vérifier l'intégrité et de caractériser la structure entière du gène cfa. A cet effet, une carte de restriction du gène cfa présent dans le plasmide pAYW19 a été réalisée avec différentes enzymes de restriction. L'analyse des tailles des fragments obtenus après restriction, électrophorèse en gel d'agarose et coloration des fragments au bromure d'éthidium a permis de dresser la carte physique de ce gène, qui est présentée sur la Figure 4. Ces résultats ont été confirmés par le séquençage du fragment en aval du site Hindll. La séquence complète et corrigée du gène cfa a été informatisé et introduite dans un logiciel (DNA strider) permettant d'étudier les sites de restriction.

### b) Construction du vecteur binaire pTDEcfa

Cet exemple décrit la construction du vecteur binaire pTDEcfa portant le une cassette d'expression du gène cfa corrigé, bordée des régions LB et RB du plasmide Ti. Ce vecteur binaire permet le transfert, par A. tumefaciens, de ladite cassette dans le génome des cellules végétales.

### i) Construction du plasmide pBSgus

Le plasmide pBSgus a été construit par clonage du fragment EcoRI-HindIII issu du vecteur binaire pTDE4 dans le vecteur pBS (pBluescript, BRL) qui ne contient que très peu de site de restriction. Le fragment EcoRI-HindII contient le promoteur p35S et le terminateur 3'nos. Ainsi le vecteur formé, pBSgus, contient les sites uniques de restriction pour les enzymes Ncol et Nrul. Ces deux enzymes permettent d'isoler le gène *gus* tout en gardant l'intégrité des séquences régulatrices (p35S et 3'nos).

### ii) Création de la cassette d'expression du gène cfa corrigé

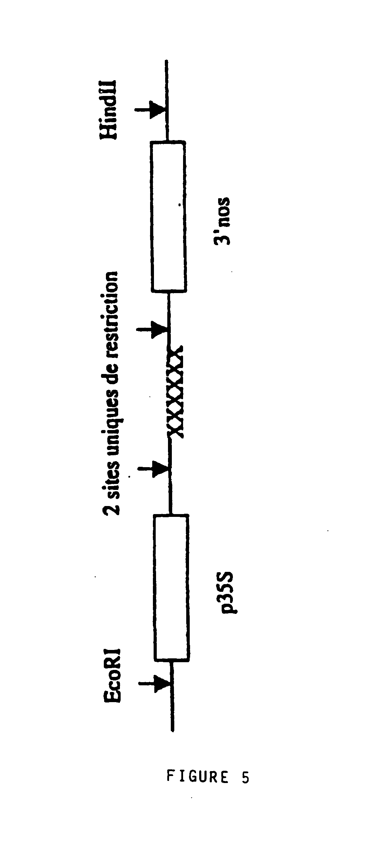

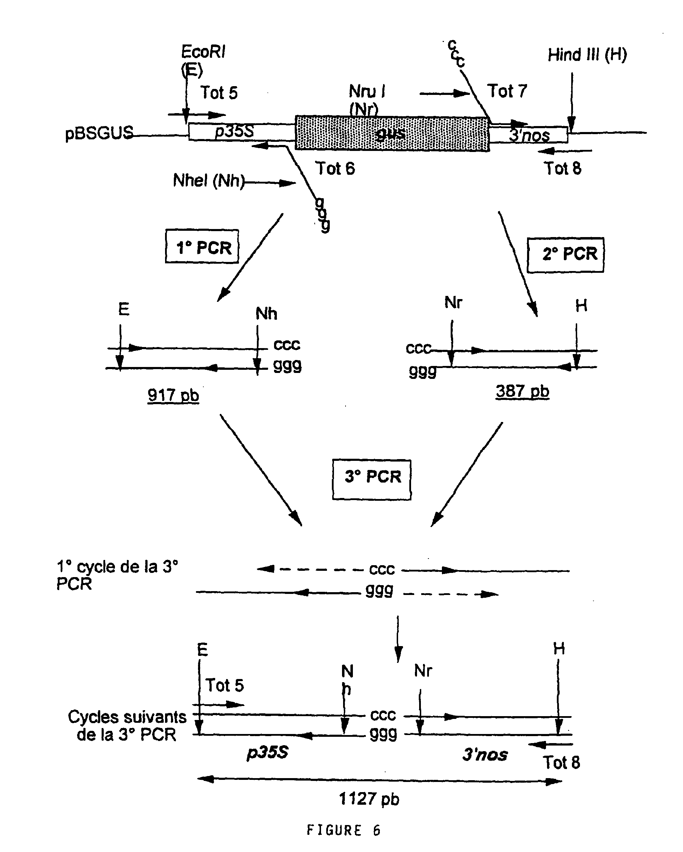

Le principe de cette étape repose sur le remplacement du gène gus du plasmide pBSgus par un site de clonage comprenant des sites uniques. Cette cassette a été créée par PCR, à partir du pBSgus, et contient le promoteur p35S et le terminateur 3'nos. Les amorces nécessaires à la PCR ont été choisies de façon à créer la structure présentée sur la Figure 5. Le choix des deux sites uniques de restriction est conditionné par la nécessité qu'ils soient absents du p35S, du 3'nos, du vecteur de clonage auquel la structure sera intégrée et du gène cfa qui sera intégré entre les 2 séquences régulatrices. Après analyse des différentes séquences, deux sites possibles ont été identifiés : Nhel et Nrul. La construction de la cassette a été réalisée comme suit (Figure 6):
- Clonage de la cassette de régulation dans le pGEM-T afin d'obtenir le vecteur pGEM(35S-3'nos)
- Création par PCR des sites Nhel en amont du gène cfa et Nrul en aval
- Clonage du produit de PCR dans le vecteur pGEM-T afin d'obtenir le vecteur pGEMcfa
- Séquençage des vecteurs pGEM(35S-3'nos) et pGEMcfa.
- Clonage du fragment NheI-NruI issu du vecteur pGEMcfa dans le vecteur pGEM(35S-3'nos) auquel on a éliminé le fragment Nhel-Nrul. Le vecteur obtenu par ce clonage est nommé pGEM(35S-cfa-3'nos).
- A partir du vecteur pGEM(35S-cfa-3'nos) il est possible d'isoler un fragment unique EcoRl-Hindll contenant la séquence (35S-cfa-3'nos).

Après optimisation des conditions de PCR pour la création de la cassette de régulation (Figure 6) celle-ci a été clonée dans le vecteur pGEM-T. Ainsi après analyse par restriction avec différentes enzymes le vecteur pGEM(35S-3'nos) est disponible dans deux clones.

De même, le vecteur pGEMcfa a été obtenu et deux clones ont été retenus.

La séquence désirée des vecteurs pGEM(35S-3'nos) et pGEMcfa a été informatisée puis introduite dans le logiciel DNAstrider. Deux clones pour chaques vecteurs ont été envoyés à la société Génome express pour être séquencés.

Les résultats du séquençage ont été obtenus pour les deux clones (2 et 3) contenant théoriquement le vecteur pGEMcfa. Après alignement avec la séquence théorique, la séquence du vecteur contenu dans le clone 2 présente plusieurs mutations et donc ce clone ne pourra pas être utilisé pour la suite du travail. La séquence du vecteur contenu dans le clone 3 contient une mutation, cependant cette mutation est une mutation dite « muette » car elle n'aura pas d'effet sur séquence de la protéine CFA

Les résultats concernant le clone 3 contenant le vecteur pGEM (35S-3'nos) ne sont pas très encourageants car le vecteur contenu dans ce clone présente plusieurs mutations. Cependant ces mutations sont relativement éloignées des séquences indispensables au bon fonctionnement du promoteur p35S et du terminateur 3'nos. Par ailleurs, le clone 4 n'a pu être séquencé. Un nouveau tri a donc été réalisé parmi les clones contenant le vecteur pGEM(35S-3'nos) et le clone 10 a été choisi pour une analyse de sa séquence.

Ainsi, le séquençage du vecteur pGEM(35S-3'nos) contenu dans le clone 10 a été réalisé. La méthode de séquençage par PCR a été utilisée avec 3 primers (T7, SP6 et un primer proche de la séquence poly CCC). La séquence obtenue a été comparée à la séquence théorique. Les 2 erreurs mises en évidence juste avant le site HinDlll semblent être des erreurs dans la séquence théorique et non du séquençage, puisqu'elles sont retrouvées dans les séquences des vecteurs pGEM(35S-3'nos) contenus dans les clones 3 et 4 précédemment séquencés.

Sur la base des résultats de séquence, le fragment Nhel-Nrul, issu du vecteur pGEMcfa contenu dans le clone 3, a été cloné dans le vecteur pGEM(35S-3'nos) contenu dans le clone 10 au niveau des sites Nhel et Nrul. Le vecteur obtenu a été désigné pGEM(35s-cfa-3'nos). Parmi les clones obtenus, un tri a été réalisé en utilisant plusieurs enzyme de restriction. Nous avons retenu le clone 13 pour la suite du clonage.

### iii) Construction du vecteur binaire

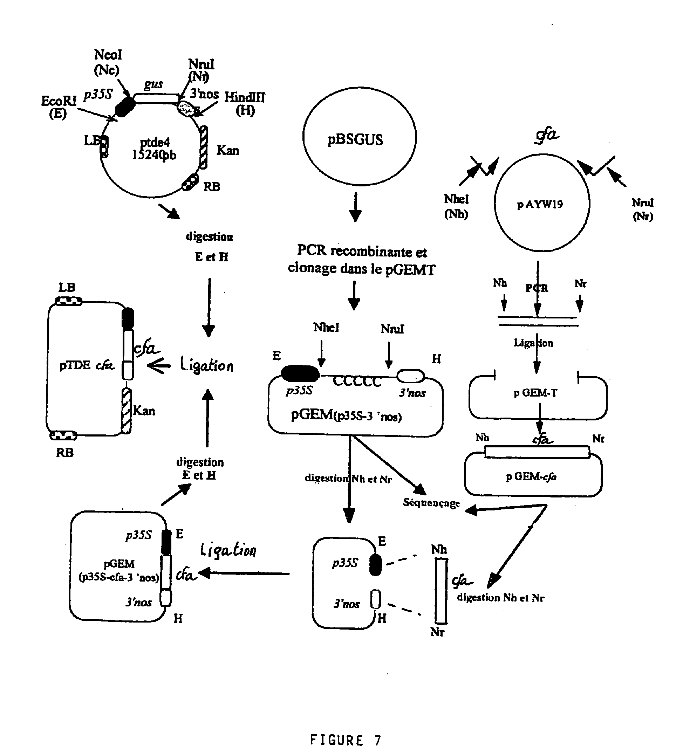

Le vecteur binaire est un vecteur comportant les séquences LB et RB du plasmide Ti, nécessaires au transfert de gènes dans les cellules végétales par Agrobactérium tumefaciens. Le vecteur binaire pTDEcfa a été construit comme suit (Figure 7):
- Clonage du fragment HindIII-EcoRI issu du vecteur pGEM(35S-cfa-3'nos)13 dans le vecteur binaire ptde4 auquel on a éliminé le fragment HindIII-EcoRI. Le vecteur obtenu est appelé pTDEcfa. Parmi les clones obtenus, un tri a été réalisé en utilisant plusieurs enzymes de restriction. Nous avons retenu le clone 8 pour effectuer la triparentale.
- Le clone contenant le vecteur pTDEcfa (clone8) a été utilisé pour préparer une quantité d'ADN qui a été testée dans une expérience de Southem-Blot. Dans cette expérience, la sonde révélant par hybridation ADN-ADN la présence du gène *cfa* a été réalisée à partir du vecteur initial pAYW19. Par cette expérience, nous avons pu vérifier en utilisant différentes enzymes de restriction que le gène *cfa* était bien intégré dans le vecteur binaire entre les 2 séquences régulatrices : promoteur et terminateur.

Le vecteur binaire a ensuite été transféré dans la bactérie A.tumefaciens, dans les conditions standard.

### ETAPE 3 : TRANSFORMATION DU VÉGÉTAL

Dans un premier temps, des protoplastes, des cellules végétales, des tissus ou des plantes de tabac sont transformées génétiquement par les acides nucléiques recombinants décrits ci-avant, par utilisation de méthodes de transfert indirect à l'aide d'un vecteur tel que les agrobactéries (par exemple *Agrobacterium tumefaciens:* C58C1Rif: pGV2260; Deblaere et al., 1985) ou en utilisant des méthodes de transformation directe (électroporation, bombardement de particules, infiltration sous vide, etc., (Kahl et Weising, 1993).

Dans le cas d'utilisation de transfert de gène indirect, les agrobactéries contiennent un plasmide possédant les régions vir (plasmide Ti désarmé) nécessaires au transfert du T-DNA dans les cellules végétales.

Dans une première étape facultative, les cellules sont transformées par un acide nucléique recombinant (figure 3) codant pour la SAM synthétase. Les transformants possédant un fort potentiel méthylant sont alors sélectionnés.

Ces lignées, ou des cellules non encore transformées, sont ensuite transformées génétiquement en vue d'insérer dans leur génome un acide nucléique recombinant contenant le gène codant pour la CFA synthase décrite précédemment.

Le protocole utilisé pour réaliser ces transformations a été décrit par Van Lijsebettens et al. (1986 J. Mol. Biol., 188, 129-145) ou répertorié dans la littérature (Plant Genetic Transformation and Gene Expression: A Laboratory Manual. Draper J., Scott R., Amritage P., et Walden R. Eds, Blackwell Scientific Publications, Oxford, 1988 ; Kahl G. Et Weiding K. (1993) Genetic engineering of plant cells. Dans Biotechnology: Genetics Fundamentals and genetic Engineering. Rehm H.J., Reed G., Pühler A. et Stadler P. Eds, VCH Publishers Inc., New York (USA) Vol. 2, pp547-659).

Dans un exemple particulier, les cellules indifférenciées de tabac variété BY2 ont été utilisées pour la transformation génétique. Ces cellules sont entretenues *in vitro* en milieu nutritif liquide. Tous les 10 jours, 10 ml de cellules sont prélevés pour inoculer 100 ml de milieu dans un erlenmeyer de 500 ml. L'erlenmeyer est placé sur un agitateur orbital à la vitesse de 140 rpm, à l'obscurité et à 24°C. La transformation a été réalisée sur des cellules âgées de 3 jours. 2ml de cellules sont prélevés et inoculés avec 100 µl d'une culture d'agrobactéries transformantes âgées de 24 h. La densité optique à λ=600 nm de la culture d'agrobactéries utilisée est ajustée à 1,5 unité. La coculture agrobactéries/cellules végétales est réalisée dans des petites boites de pétri pendant 2 jours à 24°C et à l'obscurité. Après la coculture, les cellules végétales sont abondamment rincées avec du milieu nutritif afin d'éliminer le maximun d'agrobactéries. Les cellules végétales sont ensuite mises en culture sur un milieu solide (gelifié). Ce milieu contient de la céfotaxime permettant d'éliminer le reste des agrobactéries. Il contient également de la kanamycine permettant de sélectionner les cellules végétales ayant inséré dans leur génome le fragment LB-RB du vecteur binaire et exprimant le gène conférant la résistance à la kanamycine.

Après 2 à 3 semaines de culture les cellules végétales résistantes à la kanamycine se sont développées. A ce stade une quantité de biomasse suffisante peut être prélevée pour être mise en culture en milieu liquide afin d'accélérer la production de biomasse, tout en conservant la pression de sélection (kanamycine-céfotaxime). Après plusieurs cycles de culture et pour une partie de la biomasse, nous avons éliminé la pression de sélection dans le milieu de culture afin de pouvoir comparer les profils lipidiques de cette suspension avec ceux obtenus à partir de cellules témoins cultivées dans les mêmes conditions, sans pression de sélection.

Par cette méthode, nous avons donc pu obtenir une suspension cellulaire résistante à la kanamycine. La présence du gène codant pour la CFA dans le matériel génétique de cette suspension a également été confirmée. Pour cela, nous avons réalisé des extractions d'ADN (Dellaporta et al. Plant Mol. Biol. Rep. 1 (1983) 19) et procédé à un test PCR utilisant des amorces spécifiques du gène *cfa*. Le résultat positif de la PCR montre la présence d'une bande de taille attendue correspondant au gène cfa.

Dans un autre exemple particulier, des disques foliaires de 0,5 cm de diamètre ont été préparés à partir de tabac variété SR1 cultivés *in vitro* et transformés. Brièvement, les disques ont été cultivés 24h sur un milieu solide (gelifié) de dédifférenciation cellulaire. Ils ont ensuite été inoculés par immersion dans une culture d'agrobactéries transformantes âgée de 24h et dont la densité optique λ=600 nm a été ajustée à 1 unité.

### ETAPE 4 : CULTURE ET SÉLECTION DES PLANTES TRANSFORMÉES.

Les cellules végétales transformées par les vecteurs contenant les constructions précédemment décrites sont ensuite régénérées à partir de cellules isolées, de cals ou de disques foliaires en utilisant les techniques habituelles de cultures de plantes (Plant Cell Culture: A pratical Approach. Dixon R.A. ed., IRL Press, Oxford, Washington DC., 1985). Les cellules et plantes transformées sont mises en culture suivant les procédures habituelles et décrites dans la littérature. La sélection des cellules et plantes transformées s'effectue lors de leur culture, sur milieu sélectif contenant un antibiotique ou toxine spécifiques de la cassette introduite.

Dans un exemple particulier, les disques foliaires décrits ci-avant ont été épongés sur un papier buvard stérile puis mis en coculture 2 jours à 24°C et à l'obscurité sur le même milieu solide de dédifférenciation cellulaire. Après coculture, les disques ont été rincés ½ heure dans le milieu d'induction des bourgeons, contenant de la céfotaxime et de la kanamycine. Enfin, l'induction des bourgeons a été réalisée sur ce même milieu solide. Toutes les semaines les disques sont prélevés, rincés et remis en culture sur le même milieu préparé extemporanément.

Après 4 à 5 semaines, les bourgeons qui se développent sont prélevés et mis en culture sur un milieu d'enracinement contenant céfotaxime et kanamycine

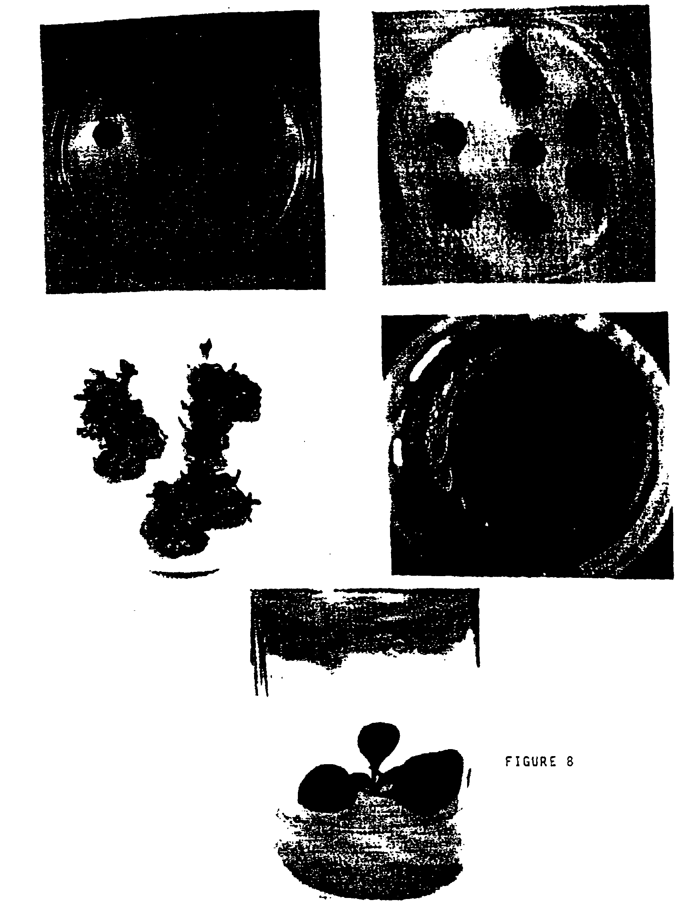

Les différentes étapes de cette transformation sont illustrées sur la Figure 8.

Une cinquantaine de bourgeons ont été obtenus par cette méthode, dont une vingtaine a été enracinnée sur milieu sélectif. Ces plantules sont multipliés par bouturage afin de produire assez de biomasse pour effectuer les vérifications PCR mettant en évidence la présence du gène *cfa* et pour extraire les lipides totaux.

### ETAPE 5 : ANALYSE DES TRANSFORMANTS

Les transformants ainsi produits sont ensuite analysés à plusieurs niveaux:
a) - Analyse directe confirmant la présence du gène dans la cellule végétale ou la plante, réalisée soit par Southern blot ou analyses PCR nécessitant des extractions d'ADN génomique. (Protocole décrit dans le Sambrook et/ou le Current Protocol).
   Sambrook J., Fritsch E.F. et Maniatis T. dans Molecular Cloning. A Laboratory Manual. Irwin N., Ford N., Nolan C. et Ferguson M. eds, Cold Spring Habor Laboratory Press. 1989.
   Current protocols in Molecular Biology. Ausubel F. M. Brent R. Kingston R.E., Moore D.D., Seidman J.G., Smith J.A. et Struhl K. eds , Current Protocols Inc Wiley, Massachusetts..., Vol. 1: Molecular Biology - Techniques. 1994 et Vol. 2: Molecular Biology - Laboratory. 1994.
b) - Analyse du produit du gène i.e. ARNm par Northern blot et confirmant la transcription du gène (Protocole décrit dans le Sambrook et/ou le Current Protocol cités ci-dessus).
c) - Analyse de la protéine synthétisée, l'enzyme (confirmant la traduction du gène en protéine) et permettant de s'assurer de la fonctionalité de celle-ci (dosage enzymatique immunoblot, Western blot).
   Le dosage de la SAM synthétase et du pool SAM dans les cellules est réalisé plus particulièrement en suivant le protocole décrit par Mathur et Sachar (1981 FEBS Lett., 287, 113-117).
   Le dosage de la CFA synthase dans les cellules et plantes de tabac est réalisé suivant le protocole décrit par Taylor et Cronan (1979 Biochemistry, 18, 3292-3300).
d) - L'analyse et l'identification des triglycérides synthétisés sont réalisées par utilisation de techniques chromatographiques de type HPLC, GC-MS ou par RMN après extraction en utilisant les protocoles décrit dans la littérature.

Ces expériences permettent de vérifier que les acides nucléiques recombinants sont fonctionnels, qu'ils peuvent être introduits dans les cellules de plantes, que lesdites cellules peuvent être régénérées, et que ces cellules produisent effectivement des acides gras ramifiés. Ces acides peuvent par la suite être récupérés directement dans les graines des plantes correspondantes, par les techniques d'extraction connues.

Dans un exemple particulier, les lipides ont été extraits des plantes obtenues ci-avant (graines, feuilles ou suspensions cellulaires) en utilisant la technique décrite par Browse et al. (Anal. Biochem. 152 (1986) 141). Les lipides obtenus ont été trans-estérifiés par la méthode de Fisher et Cherry (Lipids 18 (1983) 589). Cette méthode utilise l'hydroxyde de tétraméthyl ammonium à 20% dans le méthanol. Elle est non destructive, efficace et se prête parfaitement à la méthanolyse des huiles inhabituelles. Les esters méthyliques d'acides gras ainsi obtenus ont ensuite été analysés par chromatographie en phase gazeuse.

Les analyses lipidiques ont dans un premier temps été réalisées sur des extraits de suspensions cellulaires BY2 transformées et non transformées âgées de 7 jours et cultivées dans les mêmes conditions (milieu sans pression de sélection, agitation 140 rpm, 24°C, obscurité). Ces analyse ont permis de valider la méthode d'extraction des lipides totaux sur les suspensions cellulaires.

Par la suite, la comparaison des extraits lipidiques obtenus à partir de suspensions cellulaires de BY2 et à partir de feuilles de tabac SR1 transformées et non transformées dans différentes conditions (avec ou sans pression de sélection, avec ou sans lumière, âgées de 7 ou 14 jours), met en évidence, dans le matériel végétal transformé, la présence de composés ayant la structure d'acides gras ramifiés selon l'invention.

L'analyse en spectométrie de masse des échantillons obtenus confirme que les acides gras obtenus comprennent bien des acides gras ramifiés au sens de l'invention, c'est-à-dire possédant au moins une substitution de nature non-cyclique. Ces résultats montrent notamment la présence des acides méthyl 9 (et/ou 10) octadécanoïque et/ou méthyl 9 (et/ou 10) hexadécanoïque.

### ETAPE 6 : TRAITEMENT DES ACIDES GRAS RAMIFIES

Dans une étape facultative, les acides gras ramifiés produits par les matériels végétaux de l'invention sont traités de manière à améliorer leur propriétés physicochimiques, notamment leurs propriétés lubrifiantes.

Dans un exemple particulier, ce traitement comprend l'hydrogénation des acides gras, en vue d'augmenter la proportion d'acides gras ramifiés et ainsi d'obtenir des compositions ayant une résistance à l'oxydation accrue et des point d'ecoulement bas. Cette hydrogénation est réalisée dans les conditions connues de l'homme du métier.

### EXEMPLE B : CONSTRUCTION D'UNE CELLULE DE PLANTE GENETIQUEMENT MODIFIEE CAPABLE DE PRODUIRE DES ACIDES GRAS RAMIFIES (MULTIBRANCHES) AU COURS DE L'ELONGATION

Cet exemple illustre la construction de plantes génétiquement modifiées capables de produire des acides gras ramifiés multibranchés. En particulier, cet exemple décrit l'insertion par génie génétique dans le génome de la plante d'un gène ou d'un ensemble de gènes conduisant la plante à utiliser un Acyl-CoA non linéaire ayant un nombre d'atomes de carbone supérieur à 3 (le méthylmalonyl CoA par exemple) à la place du malonyl CoA afin d'obtenir des acides gras ramifiés dits multibranchés tels que l'acide 2, 4, 6, 8-tétraméthyl décanoïque. La synthèse des acides gras se produisant dans les chloroplastes, l'acide nucléique recombinant inséré comporte avantageusement un gène codant pour une malonyl-CoA décarboxylase (E.C. N° 4.1.1.9) de telle sorte que cette enzyme soit active au niveau chloroplastique ou bien à un autre niveau de la cellule. Ceci est obtenu soit par introduction de l'acide recombinant dans les chloroplastes, soit par insertion d'un peptide de transit permettant l'adressage de la protéine dans ce compartiment.

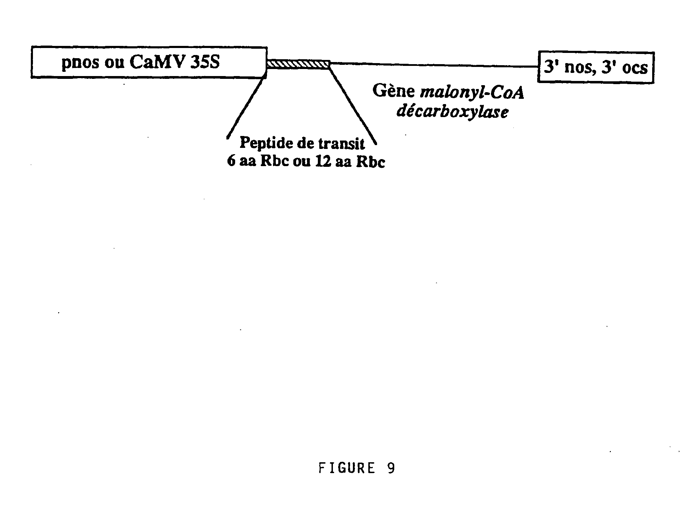

La construction de ces plantes transgéniques selon l'invention est effectuée selon les étapes suivantes (Figure 9).

### ETAPE 1 : MATÉRIEL VÉGÉTAL UTILISÉ.

Le matériel végétal utilisé est identique à celui décrit dans l'exemple A.

### ETAPE 2 : CONSTRUCTIONS GÉNIQUES.

La transformation génétique consiste à introduire une cassette d'expression comprenant le gène à transférer, le promoteur d'expression de ce gène qui pourra permettre une expression constitutive, spatiale ou temporelle et une séquence nucléotidique supplémentaire codant pour un peptide de transit permettant l'adressage de la protéine dans un compartiment cellulaire où elle sera active.

### 2.1. Sélection et isolement des acides nucléiques codant pour les enzymes.

Utilisation du gène codant pour la malonyl-CoA décarboxylase dont la séquence nucléotidique est décrite par Kolattukudy et al. (1987) ou toutes autres séquences nucléotidiques analogues provenant d'autres organismes ou microorganismes procaryotes ou eucaryotes.

Kolattukudy P.E. Rogers L.M., Poulose A.J., Jang S.H., Kim Y.S., Cheesbrough T.M. et Liggitt D.H. (1987) Developmental pattern of the expression of malonyl-CoA decarboxylase gène and the production of unique lipids in the goose uropygial glands. Arch. Biochem. Biophys., 256, 446-454.

Fernandese N.D. et Kolattukudy P.E. (1996) Cloning, sequencing and characterization of a fatty acid synthase-encoding gene from *Mycobacterium tuberculosis* var. *bovis* BCG. Gene, 170, 95-99.

Dans certains cas, des composants de la synthèse d'acides gras sont localisés dans les chloroplastes ou autres organelles. Dans notre cas, l'enzyme synthétisée : malonyl-CoA décarboxylase, doit être transportée dans les chloroplastes pour être active. Il faudra ajouter dans nos constructions géniques une séquence d'ADN codant pour un peptide de transit opérationel et reconnu par la plante réceptrice. Ces peptides de transit sont en général des séquences leader qui devront être combinées à la séquence nucléotidique du gène associé à un promoteur spécifique. Ces séquences "signal" peuvent être représentées par n'importe quelles autres séquences nucléotidiques dérivées de celle d'un gène qui s'exprime dans le cytoplasme avant d'atteindre son compartiment réactionnel approprié. L'ADN de ces peptides de transit peuvent provenir d'autres plantes. Cela peut être, par exemple, le peptide de transit de la petite sous unité de la RUBISCO du soja ou les séquences codant pour les 6 ou 12 acides aminés terminaux de la petite sous unité mature de la RUBISCO de pois et mentionnés dans le brevet de Calgene WO 94/29467 (Schéma de type 4).

### 2.2. Sélection des régions promotrices

Dans cet exemple, les promoteurs utilisés sont le promoteur 35S du virus de la mosaïque du chou fleur et le promoteur du gène de la nopaline synthase (nos). La structure de ces promoteurs a été décrite par exemple par Benfey et Chua (1990, Science, 250 959-966).

### 2.3. Sélection des régions terminatrices de la transcription

Dans cet exemple, les régions terminatrices de la transcription utilisées proviennent des gènes de la nopaline synthase (nos) et de l'octopine synthase (ocs) (Figure 9).

### 2.4. Construction des acides nucléiques recombinants (vecteurs)

Pour construire les acides nucléiques recombinant permettant d'introduire les éléments ci-dessus dans les cellules de plantes, ces éléments génétiques sont tout d'abord isolés et sous clonés dans des vecteurs de clonage de type pBR322 et pUC, qui portent des marqueurs permettant la sélection des transformants qui procurent une résistance à des agents cytotoxiques tels que antibiotiques, toxines, etc. Les cassettes d'expression sont ensuite introduites dans des vecteurs binaires contenant les séquences nécessaires à la transformation des plantes, en particulier, le vecteur T-DNA. Ce vecteur est utilisé pour la transformation des plantes et la structure des vecteurs contenant les séquences spécifiques de reconnaissance (RB et LB) a été largement décrite dans la littérature (Livre de Hoekema, the binary Plant Vector Systems, Offsetdrukkerij Kanters B.V., Alblasserdam, 1985. Molecular genetics of the Bacteria-Plant Interaction, Puhler A. Ed., Springer-Verlag, NY, 1983. Plant Genetic Transformation and Gene expression : A Laboratory Manual Draper J., Scott R., Armitage P., et Walden R. Eds, Blackwell Scientific Publications, Oxford, 1988. Kahl G. et Weising K., (1993) Genetic engineering of plant cells. Dans Biotechnology : Genetics Fundamentals and genetic Engineering. Rehm H.J., Reed G., Pühler A. et Stadler P. Eds, VCH Publishers Inc., New York (USA) Vol 2, pp 547-659).

La structure des acides nucléiques recombinants ainsi préparés est représentée sur la Figure 9.

### ETAPE 3 : TRANSFORMATION DU VÉGÉTAL

Dans un premier temps, des protoplastes, des cellules végétales, des tissus ou des plantes de tabac sont transformées génétiquement par les acides nucléiques recombinants décrits ci-avant, par utilisation de méthodes de transfert indirect à l'aide d'un vecteur tel que les agrobactéries (par exemple *Agrobacterium tumefaciens:* C58C1Rif: pGV2260; Deblaere et al., 1985) ou en utilisant des méthodes de transformation directe (éléctroporation, bombardement de particules, infiltration sous vide, etc., (Kahl et Weising, 1993).

Dans le cas d'utilisation de transfert de gène indirect, les agrobactéries contiennent un plasmide possédant les régions vir (plasmide Ti désarmé) nécessaires au transfert du T-DNA dans les cellules végétales.

Les cellules sont transformées génétiquement en vue d'insérer dans leur génome un acide nucléique recombinant (figure 9) contenant le gène codant pour la malonyl-CoA décarboxylase décrite précédemment.

Le protocole utilisé pour réaliser ces transformations a été décrit par Van Lijsebettens et al. (1986 J. Mol. Biol., 188, 129-145) ou répertorié dans la littérature (Plant Genetic Transformation and Gene Expression : A Laboratory Manual. Draper J., Scott R., Amritage P., et Walden R. Eds, Blackwell Scientific Publications, Oxford, 1988 ; Kahl G. Et Weiding K. (1993) Genetic engineering of plant cells. Dans Biotechnology: Genetics Fundamentals and genetic Engineering. Rehm H.J., Reed G., Pühler A. et Stadler P. Eds, VCH Publishers Inc., New York (USA) Vol. 2, pp547-659).

### ETAPE 4 : CULTURE ET SÉLECTION DES PLANTES TRANSFORMÉES.

Les cellules végétales transformées par les vecteurs contenant les constructions précédemment décrites sont ensuite régénérées à partir de cellules isolées, de cals ou de disques foliaires en utilisant les techniques habituelles de cultures de plantes (Plant Cell Culture : A pratical Approach. Dixon R.A. ed., IRL Press, Oxford, Washington DC., 1985). Les cellules et plantes transformées sont mises en culture suivant les procédures habituelles et décrites dans la littérature. La sélection des cellules et plantes transformées s'effectue lors de leur culture, sur milieu sélectif contenant un antibiotique ou toxine spécifiques de la cassette introduite.

### ETAPE 5 : ANALYSE DES TRANSFORMANTS

Les transformants ainsi produits sont ensuite analysés à plusieurs niveaux:
a) - Analyse directe confirmant la présence du gène dans la cellule végétale ou la plante, réalisée soit par Southern blot ou analyses PCR nécessitant des extractions d'ADN génomique selon un Protocole décrit dans :
   - Sambrook J., Fritsch E.F. et Maniatis T. dans Molecular Cloning. A Laboratory Manual. Irwin N., Ford N., Nolan C. et Ferguson M. eds, Cold Spring Habor Laboratory Press. 1989 ou,
   - Current protocols in Molecular Biology. Ausubel F. M. Brent R. Kingston R.E., Moore D.D., Seidman J.G., Smith J.A. et Struhl K. eds , Current Protocols Inc Wiley, Massachusetts..., Vol. 1: Molecular Biology - Techniques. 1994 et Vol. 2: Molecular Biology - Laboratory. 1994.
b) - Analyse du produit du gène i.e. ARNm par Northem blot et confirmant la transcription du gène (Protocole décrit dans le Sambrook et/ou le Current Protocole cités ci-dessus).
c) - Analyse de la protéine synthétisée, l'enzyme (confirmant la traduction du gène en protéine) et permettant de s'assurer de la fonctionalité de celle-ci (dosage enzymatique immunoblot, Western blot).
d) Dosage de la Malonyl-CoA décarboxylase suivant le protocole décrit par Kolattuduky et al. (1987) : Developmental pattern of the expression of Malonyl-CoA decarboxylase gene and the production of unique lipids in the goose uropygial glands. Arch. Biochem. Biophys., **256**, 446-**454**.
e) - L'analyse et l'identification des triglycérides synthétisés sont réalisées par utilisation de techniques chromatographiques de type HPLC, GC-MS ou par RMN après extraction en utilisant les protocoles décrit dans la littérature.

Ces expériences permettent de vérifier que les acides nucléiques recombinants sont fonctionnels, qu'ils peuvent être introduits dans les cellules de plantes, que lesdites cellules peuvent être régénérées, et que ces cellules produisent effectivement des acides gras ramifiés. Ces acides peuvent par la suite être récupérés directement dans les graines des plantes correspondantes, par les techniques d'extraction connues.

## Revendications

1. Procédé de production d'acides gras ramifiés, **caractérisé en ce que** lesdits acides gras ramifiés sont produits à partir d'au moins une cellule végétale ou de graines ou d'une plante , ladite cellule végétale ou lesdites graines ou ladite plante comprenant dans son génome un acide nucléique recombinant codant pour une méthyle transférase, une cyclopropane fatty acide synthase, un méthylmalonyl CoA ou une malonyl CoA décarboxylase.

2. Procédé selon la revendication 1, **caractérisé en ce qu'**il comprend en outre une étape d'extraction des acides gras ramifiés.

3. Procédé selon la revendication 2, **caractérisé en ce qu'**il comprend en outre une étape de traitement des acides gras ramifiés extraits.

4. Procédé selon l'une des revendications 1 à 3, **caractérisé en ce que** l'acide nucléique recombinant code pour un produit permettant d'induire ou de stimuler la ramification post-synthèse des acides gras produits par ladite cellule.

5. Procédé selon la revendication 4, **caractérisé en ce que** l'acide nucléique recombinant code pour une enzyme permettant le transfert d'un ou plusieurs groupements alkyl sur la ou les doubles liaisons des acides gras insaturés.

6. Procédé selon l'une des revendications 1 à 5, **caractérisé en ce que** la cellule végétale comprend en outre, un acide nucléique recombinant codant pour la SAM synthétase.

7. Procédé selon l'une des revendications 1 à 3, **caractérisé en ce que** l'acide nucléique recombinant code pour une enzyme permettant de forcer ladite cellule de plante à utiliser un substrat comportant un nombre d'atomes de carbone supérieur à 3 pour la synthèse de la chaîne aliphatique.

8. Procédé selon la revendication 7, **caractérisé en ce que** l'acide nucléique recombinant code pour une enzyme capable de forcer la plante à utiliser un acyl-CoA non linéaire.

9. Cellule de plante comprenant un acide nucléique recombinant comprenant
- une séquence d'ADN codant pour un peptide de transit opérationnel et reconnu par la plante réceptrice et permettant l'adressage de la protéine dans les chloroplastes ;
- un acide nucléique codant pour une malonyl CoA décarboxylase permettant de forcer une cellule de plante à utiliser un substrat comportant un nombre d'atomes de carbone supérieur à 3 comme substrat pour la synthèse de la chaîne aliphatique ;
- un promoteur fonctionnel dans les cellules végétales contrôlant l'expression dudit acide nucléique, et
- une région 3' de terminaison de la transcription,
ou un vecteur comprenant un tel acide nucléique recombinant.

10. Cellule de plante selon la revendication 9, **caractérisée en ce qu'**il s'agit d'une cellule de plante oléagineuse, choisie de préférence parmi le colza, le tournesol, l'arachide, le soja, le lin et le maïs.

11. Plante transgénique **caractérisée en ce qu'**elle contient au moins une cellule selon la revendication 9 ou 10.

12. Procédé de production d'acides gras ramifiés par culture cellulaire d'une cellule de plante selon l'une quelconque des revendications 9 ou 10, et comprenant :
- la culture de ces cellules dans un milieu approprié à leur croissance,
- l'extraction et la purification des acides gras ramifiés à partir des cellules ou du surnageant de ladite culture.

13. Procédé de préparation d'acides gras ramifiés à partir d'une plante transgénique dont les cellules contiennent un acide nucléique recombinant comprenant
- une séquence d'ADN codant pour un peptide de transit opérationnel et reconnu par la plante réceptrice et permettant l'adressage de la protéine dans les chloroplastes ;
- un acide nucléique codant pour une malonyl CoA décarboxylase permettant de forcer une cellule de plante à utiliser un substrat comportant un nombre d'atomes de carbone supérieur à 3 comme substrat pour la synthèse de la chaîne aliphatique ;
- un promoteur fonctionnel dans les cellules végétales contrôlant l'expression dudit acide nucléique, et
- une région 3' de terminaison de la transcription.
- la culture en champs desdites plantes transgéniques,
- la récupération des graines desdites plantes,
- l'extraction des acides gras à partir de ces graines.

14. Utilisation d'une plante transgénique selon la revendication 11, régénérée à partir d'une cellule selon l'une quelconque des revendications 9 ou 10, pour la production d'acides gras ramifiés.

15. Utilisation d'une plante ou partie d'une plante transgénique dont certaines cellules au moins comprennent un acide nucléique recombinant comprenant
- une séquence d'ADN codant pour un peptide de transit opérationnel et reconnu par la plante réceptrice et permettant l'adressage de la protéine dans les chloroplastes ;
- un acide nucléique codant pour une malonyl CoA décarboxylase permettant de forcer une cellule de plante à utiliser un substrat comportant un nombre d'atomes de carbone supérieur à 3 comme substrat pour la synthèse de la chaîne aliphatique ;
- un promoteur fonctionnel dans les cellules végétales contrôlant l'expression dudit acide nucléique, et
- une région 3' de terminaison de la transcription,
pour la production d'acides gras ramifiés.

16. Procédé de préparation d'une plante transgénique capable de produire des acides gras ramifiés, comprenant l'introduction d'un acide nucléique recombinant comprenant
- une séquence d'ADN codant pour un peptide de transit opérationnel et reconnu par la plante réceptrice et permettant l'adressage de la protéine dans les chloroplastes ;
- un acide nucléique codant pour une malonyl CoA décarboxylase permettant de forcer une cellule de plante à utiliser un substrat comportant un nombre d'atomes de carbone supérieur à 3 comme substrat pour la synthèse de la chaîne aliphatique ;
- un promoteur fonctionnel dans les cellules végétales contrôlant l'expression dudit acide nucléique, et
- une région 3' de terminaison de la transcription, dans une cellule ou partie de plante et la régénération, à partir de ces cellules ou parties de plantes d'une plante transgénique.

## Claims

1. A process for producing branched fatty acids, **characterized in that** said branched fatty acids are produced from at least one plant cell or from seeds or from a plant, the genome of said plant cell or said seeds or said plant comprising a recombinant nucleic acid coding for a methyl transferase, a cyclopropane fatty acid synthase, a methylmalonyl CoA or a malonyl CoA decarboxylase.

2. A process according to claim 1, **characterized in that** it further comprises a step of extracting branched fatty acids.

3. A process according to claim 2, **characterized in that** it further comprises a step of treating the extracted branched fatty acids.

4. A process according to one of claims 1 to 3, **characterized in that** said recombinant nucleic acid codes for a substance which allows to induce or to stimulate post-synthesis branching of fatty acids produced by said cell.

5. A process according to claim 4, **characterized in that** the recombinant nucleic acid codes for an enzyme which enables transfer of one or more alkyl groups onto the double bond or bonds of the unsaturated fatty acid.

6. A process according to one of claims 1 to 5, **characterized in that** the plant cell further comprises a recombinant nucleic acid coding for SAM synthetase.

7. A process according to one of claims 1 to 3, **characterized in that** the recombinant nucleic acid codes for an enzyme which allows to force said plant cell to use a substrate comprising more than 3 carbon atoms for synthesis of the aliphatic chain.

8. A process according to claim 7, **characterized in that** the recombinant nucleic acid codes for an enzyme which is capable of forcing the plant to use a non-linear acyl-CoA.

9. A plant cell comprising a recombinant nucleic acid comprising:
• a DNA sequence coding for a transit peptide which is operational and recognized by the receptor plant and allows addressing of the protein into the chloroplasts;
• a nucleic acid coding for a malonyl CoA decarboxylase allowing to force a plant cell to use a substrate comprising more than 3 carbon atoms as a substrate for synthesis of the aliphatic chain;
• a promoter which is functional in plant cells, controlling expression of said nucleic acid; and
• a 3'transcription termination region;
or a vector comprising said recombinant nucleic acid.

10. A plant cell according to claim 9, **characterized in that** it is a cell or an oleaginous plant, preferably selected from rapeseed, sunflower seed, peanut, soya, linseed and maize.

11. A transgenic plant, **characterized in that** it contains at least one cell in accordance with claim 9 or claim 10.

12. A process for producing branched fatty acids by cell culture of a plant cell according to claim 9 or claim 10, and comprising:
• culturing said cells in a medium which is appropriate for their growth;
• extracting and purifying branched fatty acids from the cells or from a supernatant of said culture.

13. A process for preparing branched fatty acids from a transgenic plant the cells of which contain a recombinant nucleic acid comprising:
• a DNA sequence coding for a transit peptide which is operational and recognized by the receptor plant and allows addressing of the protein into chloroplasts;
• a nucleic acid coding for a malonyl CoA decarboxylase allowing to force a plant cell to use a substrate comprising more than 3 carbon atoms as a substrate for synthesis of the aliphatic chain;
• a promoter which is functional in plant cells, controlling expression of said nucleic acid; and
• a 3' transcription termination region;
• field culturing said transgenic plants;
• recovering seeds of said plants;
• extracting fatty acids from said seeds.

14. Use of a transgenic plant according to claim 11, regenerated from a cell in accordance with claim 9 or claim 10, for the production of branched fatty acids.

15. Use of a plant or portion of a transgenic plant at least some cells of which include a recombinant nucleic acid comprising:
• a DNA sequence coding for a transit peptide which is operational and recognized by the receptor plant and allows addressing of the protein into chloroplasts;
• a nucleic acid coding for a malonyl CoA decarboxylase allowing to force a plant cell to use a substrate comprising more than 3 carbon atoms as a substrate for synthesis of the aliphatic chain;
• a promoter which is functional in plant cells, controlling expression of said nucleic acid; and
• a 3' transcription termination region;
for the production of branched fatty acids.

16. A process for preparing a transgenic plant which is capable of producing branched fatty acids, comprising introducing a recombinant nucleic acid comprising:
• a DNA sequence coding for a transit peptide which is operational and recognized by the receptor plant and allows addressing of the protein into chloroplasts;
• a nucleic acid coding for a malonyl CoA decarboxylase allowing to force a plant cell to use a substrate comprising more than 3 carbon atoms as a substrate for synthesis of the aliphatic chain;
• a promoter which is functional in plant cells, controlling expression of said nucleic acid; and
• a 3' transcription termination region;
into a cell or part of a plant and regenerating a transgenic plant from said cells or plant parts.

## Patentansprüche

1. Verfahren zur Herstellung verzweigtkettiger Fettsäuren, **dadurch gekennzeichnet, dass** die verzweigtkettigen Fettsäuren aus wenigstens einer pflanzlichen Zelle oder Samen oder einer Pflanze hergestellt werden, wobei die pflanzliche Zelle oder die Samen oder die Pflanze in ihrem Genom eine rekombinante Nukleinsäure aufweisen, die für eine Methyltransferase, eine Cyclopropanfettsäuresynthase, ein Methylmalonyl-CoA oder eine Malonyl-CoA-Decarboxylase kodiert.

2. Verfahren gemäß Anspruch 1, **dadurch gekennzeichnet, dass** es des Weiteren einen Schritt der Extraktion der verzweigtkettigen Fettsäuren aufweist.

3. Verfahren gemäß Anspruch 2, **dadurch gekennzeichnet, dass** es des Weiteren einen Schritt der Behandlung der extrahierten verzweigtkettigen Fettsäuren aufweist.

4. Verfahren gemäß einem der Ansprüche 1 bis 3, **dadurch gekennzeichnet, dass** die rekombinante Nukleinsäure für ein Produkt kodiert, das es ermöglicht, die Verzweigung nach Synthese der mittels der Zelle hergestellten Fettsäuren auszulösen oder zu stimulieren.

5. Verfahren gemäß Anspruch 4, **dadurch gekennzeichnet, dass** die rekombinante Nukleinsäure für ein Enzym kodiert, das den Transfer einer oder mehrerer Alkylgruppen auf die Doppelbindung(en) der ungesättigten Fettsäuren ermöglicht.

6. Verfahren gemäß einem der Ansprüche 1 bis 5, **dadurch gekennzeichnet, dass** die pflanzliche Zelle des Weiteren eine rekombinante Nukleinsäure aufweist, die für die SAM-Synthetase kodiert.

7. Verfahren gemäß einem der Ansprüche 1 bis 3, **dadurch gekennzeichnet, dass** die rekombinante Nukleinsäure für ein Enzym kodiert, das es ermöglicht, die Pflanzenzelle dazu zu zwingen, für die Synthese der aliphatischen Kette ein Substrat zu verwenden, das eine Anzahl Kohlenstoffatome von größer 3 aufweist.

8. Verfahren gemäß Anspruch 7, **dadurch gekennzeichnet, dass** die rekombinante Nukleinsäure für ein Enzym kodiert, das in der Lage ist, die Pflanze eine nicht lineare Acyl-CoA zu verwenden zu zwingen.

9. Pflanzenzelle mit einer rekombinanten Nukleinsäure, welche aufweist:
- eine DNA-Sequenz, die für ein Transferpeptid kodiert, das operationell ist und von der Empfängerpflanze wiedererkannt wird und das die Adressierung des Proteins in den Chloroplasten ermöglicht;
- eine Nukleinsäure, welche für eine Malonyl-CoA-Decarboxylase kodiert, die es ermöglicht, die Pflanzenzelle für die Synthese der aliphatischen Kette ein Substrat zu verwenden zu zwingen, das eine Anzahl Kohlenstoffatome von größer 3 aufweist;
- einen in den pflanzlichen Zellen funktionellen Promotor, der die Expression der Nukleinsäure kontrolliert, und
- eine 3'-Region zur Terminierung der Transkription,
oder einen Vektor, der eine derartige rekombinante Nukleinsäure aufweist.

10. Pflanzenzelle gemäß Anspruch 9, **dadurch gekennzeichnet, dass** es sich um eine Ölpflanzenzelle handelt, die vorzugsweise aus Raps, Sonnenblume, Erdnuss, Soja, Leinen und Mais gewählt ist.

11. Transgene Pflanze, **dadurch gekennzeichnet, dass** sie wenigstens eine Zelle gemäß Anspruch 9 oder 10 enthält.

12. Verfahren zu Herstellung verzweigtkettiger Fettsäuren durch Zellkultur einer Pflanzenzelle gemäß einem der Ansprüche 9 oder 10, welches aufweist:
- die Kultur von derartigen Zellen in einem Medium, das für ihr Wachstum geeignet ist;
- die Extraktion und das Reinigen der verzweigtkettigen Fettsäuren aus den Zellen oder dem Überstand der Kultur.

13. Verfahren zu Herstellung verzweigtkettiger Fettsäuren aus einer transgenen Pflanze, deren Zellen eine rekombinante Nukleinsäure aufweisen, welche aufweist:
- eine DNA-Sequenz, die für ein Transferpeptid kodiert, das operationell ist und von der Empfängerpflanze wiedererkannt wird und das die Adressierung des Proteins in den Chloroplasten ermöglicht;
- eine Nukleinsäure, welche für eine Malonyl-CoA-Decarboxylase kodiert, die es ermöglicht, die Pflanzenzelle für die Synthese der aliphatischen Kette ein Substrat zu verwenden zu zwingen, das eine Anzahl Kohlenstoffatome von größer 3 aufweist;
- einen in den pflanzlichen Zellen funktionellen Promotor, der die Expression der Nukleinsäure kontrolliert,
- eine 3'-Region zur Terminierung der Transkription,
- die Kultur der transgenen Pflanzen auf einem Feld,
- das Wiedergewinnen von Samen der Pflanzen,
- die Extraktion von Fettsäuren aus den Samen.

14. Verwendung einer transgenen Pflanze gemäß Anspruch 11, die aus einer Zelle gemäß einem der Ansprüche 9 oder 10 wiederhergestellt wurde, für die Herstellung verzweigtkettiger Fettsäuren.

15. Verwendung einer transgenen Pflanze oder eines Teils einer transgenen Pflanze, wovon bestimmte Zellen wenigstens eine rekombinante Nukleinsäure aufweisen, die aufweist:
- eine DNA-Sequenz, die für ein Transferpeptid kodiert, das operationell ist und von der Empfängerpflanze wiedererkannt wird und das die Adressierung des Proteins in den Chloroplasten ermöglicht;
- eine Nukleinsäure, welche für eine Malonyl-CoA-Decarboxylase kodiert, die es ermöglicht, die Pflanzenzelle für die Synthese der aliphatischen Kette ein Substrat zu verwenden zu zwingen, das eine Anzahl Kohlenstoffatome von größer 3 aufweist;
- einen in den pflanzlichen Zellen funktionellen Promotor, der die Expression der Nukleinsäure kontrolliert, und
- eine 3'-Region zur Terminierung der Transkription,
für die Herstellung verzweigtkettiger Fettsäuren.

16. Verfahren zur Herstellung einer transgenen Pflanze, die in der Lage ist, verzweigtkettige Fettsäuren herzustellen, welches das Einbringen einer rekombinanten Nukleinsäure aufweist, die umfasst:
- eine DNA-Sequenz, die für ein Transferpeptid kodiert, das operationell ist und von der Empfängerpflanze wiedererkannt wird und das die Adressierung des Proteins in den Chloroplasten ermöglicht;
- eine Nukleinsäure, welche für eine Malonyl-CoA-Decarboxylase kodiert, die es ermöglicht, die Pflanzenzelle für die Synthese der aliphatischen Kette ein Substrat zu verwenden zu zwingen, das eine Anzahl Kohlenstoffatome von größer 3 aufweist;
- einen in den pflanzlichen Zellen funktionellen Promotor, der die Expression der Nukleinsäure kontrolliert, und
- eine 3'-Region zur Terminierung der Transkriptase,
in einer Zelle oder einem Teil einer Pflanze sowie das Wiederherstellen einer transgenen Pflanze aus den Zellen oder Teilen der Pflanze.
